(19)

Europäisches
Patentamt

European
Patent Office

Office européen
des brevets

(11) **EP 2 317 472 A1**

(12) **EUROPEAN PATENT APPLICATION**

(43) Date of publication:
**04.05.2011 Bulletin 2011/18**

(51) Int Cl.:
*G06T 7/00* (2006.01)

(21) Application number: **10186750.5**

(22) Date of filing: **06.10.2010**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **13.10.2009 KR 20090097003**

(71) Applicant: **MEDISON CO., LTD.**
**Gangwon-do 250-875 (KR)**

(72) Inventor: **Lee, Kwang Hee**
**135-851, Seoul (KR)**

(74) Representative: **Schmid, Wolfgang**
**Lorenz & Kollegen**
**Patentanwälte Partnerschaftsgesellschaft**
**Alte Ulmer Strasse 2**
**89522 Heidenheim (DE)**

(54) **An ultrasound system generating an image based on brightness value of data**

(57) An ultrasound system extracts label regions from ultrasound data based on image brightness. An ultrasound data acquisition unit forms ultrasound data of a target object. A processing unit is connected to the ultrasound data acquisition unit. The processing unit forms volume data including a plurality of voxels based on the ultrasound data, and extracts label regions having lower brightness values than a reference value from the volume data to thereby form an ultrasound image by rendering the extracted label regions.

## FIG. 1

**Description**

CROSS-REFERENCE TO RELATED APPLICATIONS

The present application claims priority from Korean Patent Application No. 10-2009-0097003 filed on October 13. 2009, the entire subject matter of which is incorporated herein by reference.

TECHNICAL FIELD

The present invention generally relates to ultrasound systems, and more particularly to an ultrasound system that generates an image based on brightness value of data.

BACKGROUND

An ultrasound system has become an important and popular diagnostic tool due to its non-invasive and non-destructive nature. The ultrasound system can provide high dimensional real-time ultrasound images of inner parts of target objects without a surgical operation.
The ultrasound system transmits ultrasound signals to the target objects, receives echo signals reflected from the target objects and provides two or three-dimensional ultrasound images of the target objects based on the echo signals.
Conventionally, it may be diagnosed as polycystic ovary syndrome (PCOS) by observing the ultrasound images of the target objects. Therefore, an automated detecting system for detecting specified region of the target objects based on the brightness value of target object images has been required.

SUMMARY

An embodiment for extracting a region based on image intensity is disclosed herein. In one embodiment, by way of non-limiting example, an ultrasound system includes an ultrasound data acquisition unit configured to form ultrasound data of a target object; and a processing unit connected to the ultrasound data acquisition unit. The processing unit is configured to form volume data including a plurality of voxels based on the ultrasound data, and extract label regions having lower brightness values than a reference value from the volume data to thereby form an ultrasound image by rendering the extracted label regions.
In another embodiment, a method of extracting an object of interest based on brightness value includes forming ultrasound data of a target object; forming volume data including a plurality of voxels based on the ultrasound data; extracting label regions having lower brightness values than a reference value from the volume data; and forming a three-dimensional ultrasound image by rendering the extracted label regions.
This Summary is provided to introduce a selection of concepts in a simplified form that are further described below in the Detailed Description. This Summary is not intended to identify key or essential features of the claimed subject matter, nor is it intended to be used in determining the scope of the claimed subject matter.

BRIEF DESCRIPTION OF THE DRAWINGS

FIG. 1    is a block diagram showing an illustrative embodiment of an ultrasound system.
FIG. 2    is a block diagram showing an illustrative embodiment of an ultrasound data acquisition unit in FIG. 1.
FIG. 3    is a schematic diagram showing a plurality of frames of the three-dimensional ultrasound image.
FIG. 4    is a flowchart showing a detection process to identify an object of interest of the target object based on a voxel brightness value.
FIG. 5    is a schematic diagram showing an example of volume data.
FIG. 6    is a schematic diagram showing an example of label regions.
FIG. 7    is a schematic diagram showing an example of a seed volume and a boundary of a label region.
FIG. 8    is a flowchart showing a detection process to identify an object of interest of the target object based on pixel brightness value.
FIG. 9    is a schematic diagram showing an example of a seed point and a boundary of a label region.

DETAILED DESCRIPTION

This detailed description is provided with reference to the accompanying drawings. One of ordinary skill in the art may realize that the following description is illustrative only and is not in any way limiting. Other embodiments of the present invention may readily suggest themselves to such skilled persons having the benefit of this disclosure.

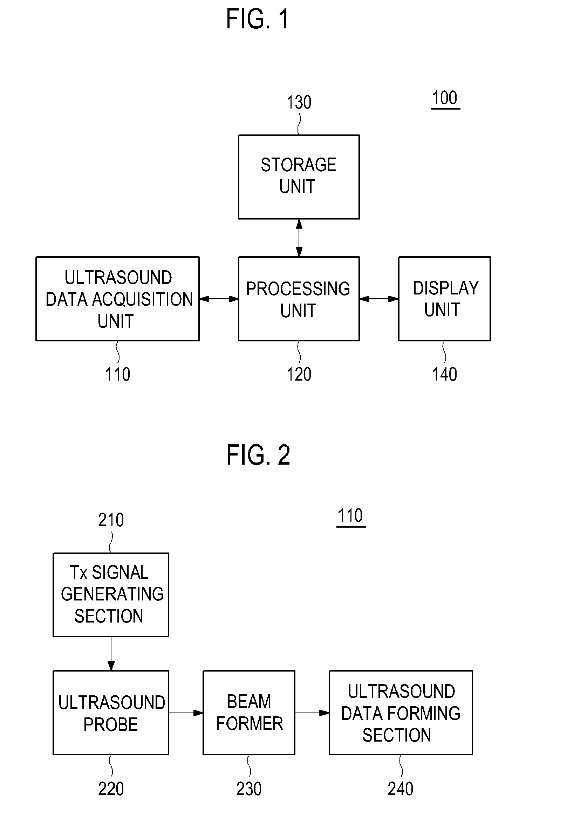

Referring to FIG. 1, an ultrasound system 100 in accordance with an illustrative embodiment is shown. As depicted therein, the ultrasound system 100 may include an ultrasound data acquisition unit 110. The ultrasound data acquisition unit 110 may be configured to transmit and receive ultrasound signals to and from a target object to thereby output ultrasound data.

FIG. 2 is a block diagram showing an illustrative embodiment of the ultrasound data acquisition unit 110. Referring to FIG. 2, the ultrasound data acquisition unit 110 may include a transmit (Tx) signal generating section 210, an ultrasound probe 220, a beam former 230 and an ultrasound data forming section 240.

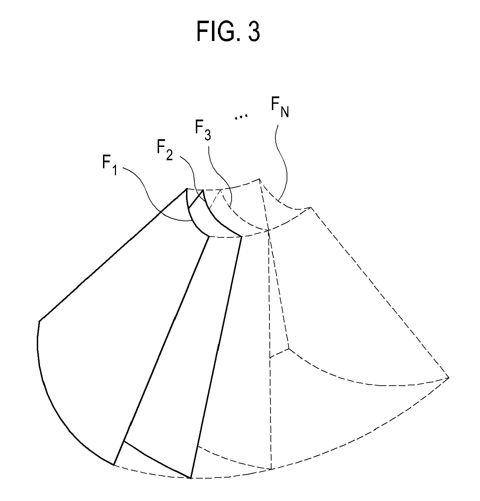

The Tx signal generating section 210 may be configured to generate Tx signals. The Tx signal generating section 210 may generate the Tx signals at a predetermined time to thereby form a plurality of Tx signals corresponding to a plurality of frames $F_i$ ($1 \leq i \leq N$) representing the target object, as shown in FIG. 3. The frames may include a brightness mode (B mode) image. However, it should be noted herein that the frames may not be limited thereto.

FIG. 3 is a schematic diagram showing an example of acquiring ultrasound data corresponding to the plurality of frames $F_i$ ($1 \leq i \leq N$). The plurality of frames $F_i$ ($1 \leq i \leq N$) may represent sectional planes of the target object (not shown).

Referring back to FIG. 2, the ultrasound probe 220 may include a plurality of elements (not shown) for reciprocally converting between ultrasound signals and electrical signals. The ultrasound probe 220 may be configured to transmit ultrasound signals to the target object in response to the Tx signals provided from the Tx signal generating section 210. The ultrasound probe 220 may further receive ultrasound echo signals reflected from the target object to thereby output the received signals. The received signals may be analog signals. The ultrasound probe 220 may include a three-dimensional (3D) mechanical probe, a two-dimensional (2D) array probe and the like. However, it should be noted herein that the ultrasound probe 220 may not be limited thereto.

The beam former 230 may be configured to convert the received signals provided from the ultrasound probe 220 into digital signals. The beam former 230 may further apply delays to the digital signals in consideration of distances between the elements and focal points to thereby output digital receive-focused signals.

The ultrasound data forming section 240 may be configured to form ultrasound data corresponding to each of the plurality of frames $F_i$ ($1 \leq i \leq N$) based on the digital receive-focused signals provided from the beam former 230. The ultrasound data may be radio frequency (RF) data. However, it should be noted herein that the ultrasound data may not be limited thereto. The ultrasound data forming section 240 may further perform various signal processing (e.g., gain adjustment) to the digital receive-focused signals.

Referring back to FIG. 1, a processing unit 120 is connected to the ultrasound data acquisition unit 110.

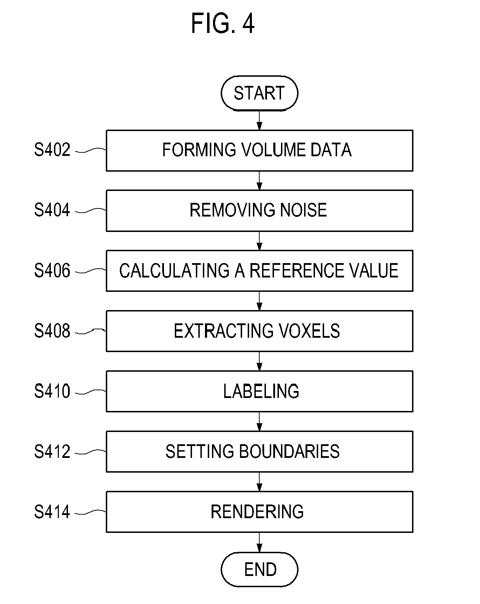

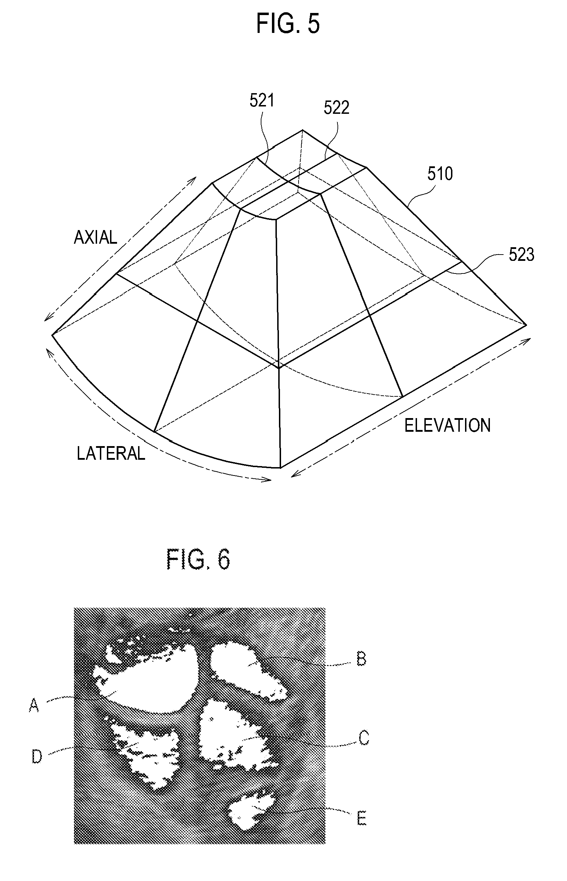

FIG. 4 is a flowchart showing a detection process for detecting an object of interest in a target object, i.e. a cyst, based on voxel brightness value. Referring to FIG. 4, the processing unit 120 may be configured to synthesize the plurality of ultrasound data corresponding to the plurality of frames $F_i$ ($1 \leq i \leq N$) to thereby form volume data 510 as shown in FIG. 5, at step S402. The volume data 510 may be stored in a storage unit 130 as shown in FIG. 1.

FIG. 5 is a schematic diagram showing an example of the volume data 510. The volume data 510 may include a plurality of voxels (not shown) having brightness values. In FIG. 5, reference numerals 521 to 523 represent an A plane, a B plane and a C plane. The A plane 521, the B plane 522 and the C plane 523 may be mutually orthogonal. Also, in FIG. 5, the axial direction may be a Tx direction of the ultrasound signals, the lateral direction may be a longitudinal direction of the elements, and the elevation direction may be a swing direction of the elements, i.e., a depth direction of a 3D ultrasound image.

Referring back to FIG. 4, the processing unit 120 may remove noise from the volume data, at step s404. In one embodiment, the processing unit 120 may employ a total variation filtering method, which is to minimize a total variation energy function.

The total variation energy function may be defined as the following equation.

$$E_{\mathrm{TV}} = \int_{\Omega} \| \nabla u \| \, d\Omega \ \ \text{with constraint} \ \ \frac{1}{|\Omega|} \int_{\Omega} \frac{(u - u_o)^2}{u} \, d\Omega = \sigma_n^{\,2} \tag{1}$$

wherein "$\Omega$" denotes dimension of the volume data, "$u$" denotes the volume data with the noise removed, "$u_0$" denotes a volume data function having the noise, and "$\sigma_n$" denotes differences between the volume data with the noise removed and the volume data having the noise.

The Euler Lagrange equation may be reduced to the following equation.

$$\frac{\partial u}{\partial t} = div(F) - \lambda\left(\frac{u^2 - u_0^2}{u}\right), \, in\,\Omega \quad (2)$$

wherein "F" denotes a force term derived from the Euler Lagrange equation, "div(F)" denotes a divergence of the "F", and "$\lambda$" denotes a weight constant.

Equation (2) may be reduced to equation (3) for minimizing of the total variation energy function of equation (1). The minimizing of the total variation energy function may denote calculation of a value for minimizing the total variation energy function.

$$\lambda = \frac{1}{\sigma_n^2 |\Omega|} \int \left(\frac{u - u_0}{u + u_0} u\right) div(F) d\Omega \quad (3)$$

Equation (3) may represent the updated equation for obtaining the volume data with the noise removed "u" by iterating the equation (2) with the passage of time.

In equation (2) and (3), the volume data with the noise removed "u" may be acquired by substituting the force term "F" with $\dfrac{\nabla u}{\|\nabla u\|}$ to apply the total variation filtering method only. In other words, the volume data with the noise removed "u" may be acquired by minimizing the total variation energy function within a predetermined range of $\sigma_n$.

In another embodiment, the processing unit 120 may apply filtering methods among various noise removing filtering methods.

The processing unit 120 may calculate first reference value ($T_{global}$) for extracting voxels having specific brightness value from the noise removed volume data, at step S406. In one embodiment, the processing unit 120 may calculate the first reference value using the equation (4).

$$T_{global} = \frac{1}{N} \sum_n I(n) - \sigma, \quad 0 \le n \le N - 1 \quad (4)$$

wherein "N" denotes the number of voxels included in the volume data, "I(n)" denotes the brightness value of the $n^{th}$ voxel, and "$\sigma$" denotes the brightness value standard deviation of all the voxels in the volume data.

The processing unit 120 may extract voxels having a specific brightness value based on the calculated first reference value, at step S408. In one embodiment, the processing unit 120 may extract voxels having a lower value than the first reference value by comparing the voxel brightness value with the first reference value.

The processing unit 120 may label the extracted voxels to set at least one of the label regions, at step S410. In one embodiment, the processing unit 120 may set values of voxels having a lower brightness value than the first reference value as "1" and set values of voxels having a higher brightness value than the first reference value as "0". Neighboring voxels having a value of "1" are set as the same label region. Referring to FIG. 6, the extracted voxels may be set as label regions identified as A, B, C, D and E to be distinguished from each other.

The set label regions may be set narrower or wider than real region of the object of interest. Therefore, the processing unit 120 may set boundaries of each label region, at step S412.

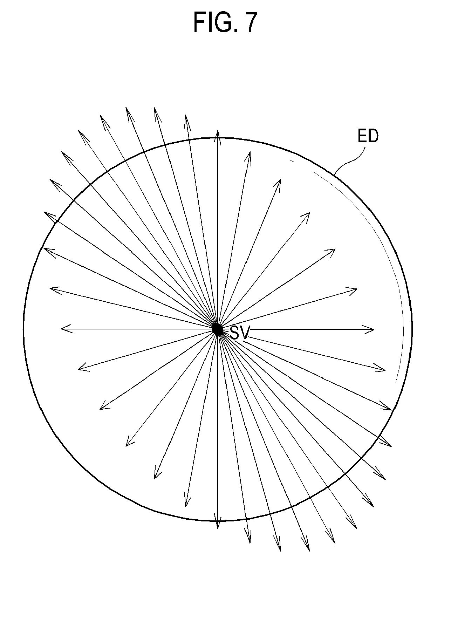

In one embodiment, the processing unit 120 may extract a middle point of the label region ED as depicted in FIG. 7 and set the middle point as a seed volume (SV). The processing unit 120 may set the boundaries of the label regions using the active contour algorithm based on the SV. In this case, the processing unit 120 may enlarge the SV radially. The processing unit 120 may stop the enlargement of the SV when the difference between the brightness values of the voxels within the SV and the brightness values of the voxels outside the SV becomes greater than a critical value to thereby extract the boundary of the label region ED.

The processing unit 120 may perform rendering on the volume data of the label region having the boundary to thereby form a three-dimensional ultrasound image of the label region, at step S414. The rendering may include a surface rendering, volume rendering and the like.

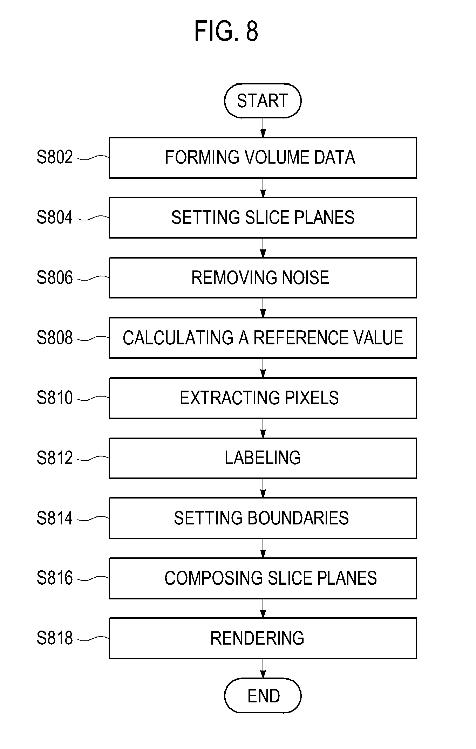

FIG. 8 is a flowchart showing a detection process of an object of interest of the target object based on pixel brightness value. Referring to FIG. 8, the processing unit 120 may form the volume data 510 as shown in FIG. 5 based on a plurality of ultrasound data transmitted from the ultrasound data acquisition unit 110, at step S802.

The processing unit 120 may set a plurality of slice planes on the volume data, at step S804. In one embodiment, the processing unit 120 may set a reference slice plane on the volume data 510. The reference slice plane may include one of three slice planes: A plane, B plane or C plane as shown in FIG. 5. The reference slice plane is not limited thereto. The processing unit 120 may set a plurality of slice planes parallel to the reference slice plane. Each slice plane may include a plurality of pixels having brightness values.

The processing unit 120 may perform a noise removing operation on each slice plane to thereby remove noise from each slice plane, at step S806. The noise removing method is the same as above, so a detailed description of the noise removing operation is omitted.

The processing unit 120 may calculate a second reference value for extracting pixels having a specific brightness value from the noise removed slice planes, at step S808. The second reference value may be calculated using equation (4) as previously described, so a detailed description of a method for calculating the second reference value is omitted.

The processing unit 120 may extract pixels having a specific brightness value from the noise removed slice planes based on the calculated second reference value, at step S810. In one embodiment, the processing unit 120 may extract pixels having lower value than the second reference value by comparing the pixel brightness value with the second reference value.

The processing unit 120 may labelthe extracted pixels of each slice plane to set label regions, at step S812. In one embodiment, the processing unit 120 may set values of the pixels having lower brightness value than the second reference value as "1" and set values of the pixels having higher brightness value than the second reference value as "0". Neighboring pixels having a value of "1" are set as the same label region.

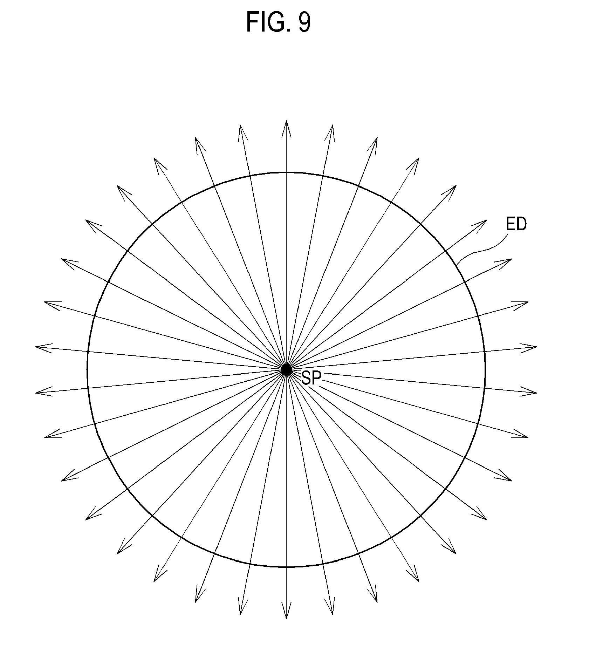

The processing unit 120 may set boundaries of each label region on each slice plane, at step S814. In one embodiment, the processing unit 120 may extract a middle point of each label region as depicted in FIG. 9 and set the extracted middle point as a seed point (SP). The processing unit 120 may set the boundaries of the label regions using the active contour algorithm based on the SP. In other words, the processing unit 120 may enlarge the SP radially. The processing unit 120 may stop the enlargement of the SP when the difference between the brightness values of the pixels within the SV and the brightness values of the voxels outside the SV becomes greater than a critical value to thereby extract the boundaries of the label region ED.

The processing unit 120 may synthesize the slice planes having the label regions to thereby form the volume data, at step S816. The volume data may include label regions having volume.

The processing unit 120 may perform a rendering act using the volume data of the synthesized slice plains to thereby form a three-dimensional ultrasound image of the label regions, at step S818. The rendering act may include a surface rendering, volume rendering and the like.

Referring back to FIG. 1, the storage unit 130 may store the volume data formed by the processing unit 120. The display unit 140 may display the three-dimensional ultrasound image formed by the processing unit 120. The display unit 140 may include a cathode ray tube (CRT) display, a liquid crystal display (LCD), organic light emitting diodes (OLED) display and the like.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1.   An ultrasound system, comprising:

an ultrasound data acquisition unit configured to form ultrasound data of a target object; and
a processing unit connected to the ultrasound data acquisition unit, the processing unit being configured to form volume data including a plurality of voxels based on the ultrasound data, and extract label regions having lower brightness values than a reference value from the volume data to thereby form an ultrasound image by rendering the extracted label regions.

**2.** The ultrasound system of Claim 1, wherein the processing unit is further configured to:

calculate the reference value for detecting the label regions from the volume data;
extract voxels having lower value than the reference value by comparing the brightness value of each voxel with the reference value;
label the extracted voxels to thereby set the label regions; and
set boundaries of the label regions.

**3.** The ultrasound system of Claim 2, wherein the processing unit is further configured to:

extract a middle point of the boundary set at each label region;
set the extracted middle point as a seed volume; and
enlarge the seed volume radially to thereby set the boundary of the label region.

**4.** The ultrasound system of Claim 1, wherein the processing unit is further configured to:

set a plurality of slice planes including a plurality of pixels on the volume data;
calculate a reference value for detecting the label regions from each slice plane;
extract pixels having a lower value than the reference value by comparing the brightness value of each pixel with the reference value;
label the extracted pixels to thereby set the label regions; and
synthesize the plurality of slice planes having the label regions to thereby form the volume data.

**5.** The ultrasound system of Claim 4, wherein the processing unit is further configured to:

extract middle points of the label regions on each slice plane;
set the extracted middle points as seed points; and
enlarge the seed points radially to thereby set the boundary of each slice plane.

**6.** A method of extracting an object of interest based on brightness value, the method comprising:

forming ultrasound data of a target object;
forming volume data comprising a plurality of voxels based on the ultrasound data;
extracting label regions having lower brightness values than a reference value from the volume data; and
forming an ultrasound image by rendering the extracted label regions.

**7.** The method of Claim 6, wherein extracting label regions comprises:

calculating the reference value for detecting the label regions from the volume data;
extracting voxels having a lower value than the reference value by comparing the brightness value of each voxel with the reference value;
labeling the extracted voxels to thereby set the label regions; and
setting boundaries of the label regions.

**8.** The method of Claim 7, wherein setting boundaries comprises:

extracting a middle point of the boundary set at each label region;
setting the extracted middle point as a seed volume; and
enlarging the seed volume radially to thereby set the boundary of the label region.

**9.** The method of Claim 6, wherein extracting label regions comprises:

6

setting a plurality of slice planes comprising a plurality of pixels on the volume data;
calculating a reference value for detecting the label regions from each slice plane;
extracting pixels having a lower value than the reference value by comparing the brightness value of each pixel with the reference value;
labeling the extracted pixels to thereby set the label regions; and
synthesizing the plurality of slice planes having the label regions to thereby form the volume data.

10. The method of Claim 9, wherein labeling the extracted pixels comprises:

extracting middle points of the label regions on each slice plane;
setting the extracted middle points as seed points; and
enlarging the seed points radially to thereby set the boundary of each slice plane.

# FIG. 1

130

100

STORAGE
UNIT

ULTRASOUND
DATA ACQUISITION
UNIT

PROCESSING
UNIT

DISPLAY
UNIT

110

120

140

# FIG. 2

210

110

Tx SIGNAL
GENERATING
SECTION

ULTRASOUND
PROBE

BEAM
FORMER

ULTRASOUND
DATA FORMING
SECTION

220

230

240

## FIG. 3

# FIG. 4

```
                    ( START )
                        |
                        v
S402 ─┐   ┌──────────────────────────────┐
      └──►│     FORMING VOLUME DATA       │
          └──────────────────────────────┘
                        |
                        v
S404 ─┐   ┌──────────────────────────────┐
      └──►│       REMOVING NOISE          │
          └──────────────────────────────┘
                        |
                        v
S406 ─┐   ┌──────────────────────────────┐
      └──►│  CALCULATING A REFERENCE VALUE │
          └──────────────────────────────┘
                        |
                        v
S408 ─┐   ┌──────────────────────────────┐
      └──►│      EXTRACTING VOXELS        │
          └──────────────────────────────┘
                        |
                        v
S410 ─┐   ┌──────────────────────────────┐
      └──►│          LABELING             │
          └──────────────────────────────┘
                        |
                        v
S412 ─┐   ┌──────────────────────────────┐
      └──►│      SETTING BOUNDARIES       │
          └──────────────────────────────┘
                        |
                        v
S414 ─┐   ┌──────────────────────────────┐
      └──►│          RENDERING            │
          └──────────────────────────────┘
                        |
                        v
                    (  END  )
```

# FIG. 5

# FIG. 6

## FIG. 7

# FIG. 8

START

S802 — FORMING VOLUME DATA

S804 — SETTING SLICE PLANES

S806 — REMOVING NOISE

S808 — CALCULATING A REFERENCE VALUE

S810 — EXTRACTING PIXELS

S812 — LABELING

S814 — SETTING BOUNDARIES

S816 — COMPOSING SLICE PLANES

S818 — RENDERING

END

# FIG. 9

Europäisches
Patentamt

European
Patent Office

Office européen
des brevets

# EUROPEAN SEARCH REPORT

Application Number

EP 10 18 6750

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/267499 A1 (DEISCHINGER HARALD [AT] ET AL) 30 October 2008 (2008-10-30)<br>* figures 1,6,8 *<br>* paragraphs [0020] - [0022] *<br>* paragraphs [0035] - [0036] *<br>* paragraphs [0005], [0040] *<br>----- | 1-10 | INV.<br>G06T7/00 |
| X | CHANG R-F ET AL: "Segmentation of breast tumor in three-dimensional ultrasound images using three-dimensional discrete active contour model",<br>ULTRASOUND IN MEDICINE AND BIOLOGY, NEW YORK, NY, US,<br>vol. 29, no. 11,<br>1 November 2003 (2003-11-01), pages 1571-1581, XP004477170,<br>ISSN: 0301-5629, DOI:<br>DOI:10.1016/S0301-5629(03)00992-X<br>* abstract *<br>* figures 1,4,7,8 *<br>* p. 1575 section "Automatic thresholding" *<br>----- | 1,6 | |
| X,P | EP 2 130 497 A1 (MEDISON CO LTD [KR]; KOREA ADVANCED INST SCI & TECH [KR]) 9 December 2009 (2009-12-09)<br>* abstract *<br>* figure 6 *<br>* paragraph [0024] *<br>----- | 1,6 | TECHNICAL FIELDS SEARCHED (IPC)<br><br>G06T<br>G06K |

-/--

The present search report has been drawn up for all claims

| Place of search | Date of completion of the search | Examiner |
|---|---|---|
| Munich | 10 January 2011 | Scholz, Volker |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................

& : member of the same patent family, corresponding
document

EPO FORM 1503 03.82 (P04C01)

Europäisches
Patentamt

European
Patent Office

Office européen
des brevets

# EUROPEAN SEARCH REPORT

**Application Number**

EP 10 18 6750

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | COHEN L D ET AL: "FINITE-ELEMENT METHODS FOR ACTIVE CONTOUR MODELS AND BALLOONS FOR 2-D AND 3-D IMAGES", IEEE TRANSACTIONS ON PATTERN ANALYSIS AND MACHINE INTELLIGENCE, IEEE SERVICE CENTER, LOS ALAMITOS, CA, US, vol. 15, no. 11, 1 November 1993 (1993-11-01), pages 1131-1147, XP000413105, ISSN: 0162-8828, DOI: DOI:10.1109/34.244675 * abstract * * section II. A+B * | 5,10 | |
| A | EP 2 098 993 A1 (MEDISON CO LTD [KR]) 9 September 2009 (2009-09-09) * abstract * * figures 1,4,5,6 * * paragraph [0017] - paragraph [0024] * | 1-10 | |
| A | CHEN SHENG ET AL: "Total Variation-Based Speckle Reduction Using Multi-grid Algorithm for Ultrasound Images", 1 January 2005 (2005-01-01), IMAGE ANALYSIS AND PROCESSING - ICIAP 2005 LECTURE NOTES IN COMPUTER SCIENCE;;LNCS, SPRINGER, BERLIN, DE, PAGE(S) 245 - 252, XP019018769, ISBN: 978-3-540-28869-5 * abstract * * section 2 * | 1-10 | TECHNICAL FIELDS SEARCHED (IPC) |

The present search report has been drawn up for all claims

| Place of search | Date of completion of the search | Examiner |
|---|---|---|
| Munich | 10 January 2011 | Scholz, Volker |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**    EP 10 18 6750

This annex lists the patent family members relating to the patent documents cited in the above-mentioned European search report.
The members are as contained in the European Patent Office EDP file on
The European Patent Office is in no way liable for these particulars which are merely given for the purpose of information.

10-01-2011

| Patent document cited in search report | | Publication date | Patent family member(s) | | Publication date |
|---|---|---|---|---|---|
| US 2008267499 | A1 | 30-10-2008 | NONE | | |
| EP 2130497 | A1 | 09-12-2009 | JP | 2009291618 A | 17-12-2009 |
| | | | KR | 20090127100 A | 09-12-2009 |
| | | | US | 2009306507 A1 | 10-12-2009 |
| EP 2098993 | A1 | 09-09-2009 | JP | 2009207899 A | 17-09-2009 |
| | | | KR | 20090095150 A | 09-09-2009 |
| | | | US | 2009227869 A1 | 10-09-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020090097003